(19) **Europäisches Patentamt / European Patent Office / Office européen des brevets**

(11) **EP 4 714 424 A1**

(12) # EUROPEAN PATENT APPLICATION
published in accordance with Art. 153(4) EPC

(43) Date of publication:
**25.03.2026 Bulletin 2026/13**

(21) Application number: **24807245.6**

(22) Date of filing: **15.05.2024**

(51) International Patent Classification (IPC):
**A61K 8/29** (2006.01)    **A61Q 17/04** (2006.01)
**C01G 23/053** (2006.01)

(52) Cooperative Patent Classification (CPC):
**A61K 8/29; A61Q 17/04; C01G 23/053**

(86) International application number:
**PCT/JP2024/018031**

(87) International publication number:
**WO 2024/237295 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 16.05.2023 JP 2023080851
30.11.2023 JP 2023202872

(71) Applicant: **Tayca Corporation**
**Osaka-shi,**
**Osaka 551-0022 (JP)**

(72) Inventors:
• **SHIBATA Kazuya**
**Osaka-shi, Osaka 551-0022 (JP)**
• **KAYAHARA Kenji**
**Osaka-shi, Osaka 551-0022 (JP)**

(74) Representative: **Kador & Partner Part mbB**
**Corneliusstraße 15**
**80469 München (DE)**

(54) **POWDER COMPOSITION AND COSMETIC USING SAME**

(57)    Provided is a powder composition which is capable of easily obtaining a dispersion and has high dispersibility. The powder composition includes: titanium oxide powder as powder to be treated; at least one hydrophobic treatment agent selected from the group consisting of a fatty acid, silicone, and a silane coupling agent; a surfactant; and an oil agent, a content of the surfactant is 0.1% or more and 50% or less of a weight of the powder to be treated, and a content of the oil agent is 0.1% or more and 50% or less of the weight of the powder to be treated.

# EP 4 714 424 A1

**Description**

TECHNICAL FIELD

The present invention relates generally to a powder composition of a titanium oxide and, in particular, to a powder composition of a titanium oxide which is included in a cosmetic and a cosmetic using the powder composition.

BACKGROUND ART

In recent years, O/W type emulsified cosmetics which exhibit fresh feeling and are excellent in use feeling tend to be preferred. The organic ultraviolet absorber which has been often used so far has tendency not to be favored in recent years due to safety and the influence on the marine environment, and in order to achieve both use feeling of the O/W type emulsified cosmetics and ultraviolet ray protective effect in a compatible manner, the technology in which an inorganic scattering agent which has been subjected to water repellency treatment is dispersed in an inner oil phase is under examination. For example, a method in which an inorganic scattering agent which is surface-treated by a silane coupling agent or silicone and is excellent in dispersibility is dispersed in an inner oil phase of an O/W type emulsified cosmetic and a preparation is thereby prepared is general.

For example, described in Japanese Patent Application Laid-Open Publication No. 2009-102236 (Patent Literature 1) is an O/W type emulsified composition in which hydrophobic inorganic powder such as a titanium oxide and a zinc oxide which is hydrophobized by a hydrophobic treatment agent such as silicones or a higher fatty acid is dispersed in its oil phase.

In addition, for example, described in Japanese Patent Application Laid-Open Publication No. 2010-215602 (Patent Literature 2) is an O/W type sunscreen cosmetic which contains a zinc oxide and/or a titanium oxide and silicone oil.

CITATION LIST

PATENT LITERATURE

Patent Literature 1: Japanese Patent Application Laid-Open Publication No. 2009-102236
Patent Literature 2: Japanese Patent Application Laid-Open Publication No. 2010-215602

SUMMARY OF THE INVENTION

TECHNICAL PROBLEM

In a case in which the inorganic scattering agent is dispersed in the inner oil phase of the O/W type emulsified cosmetic, in order to obtained fresh use feeling, it is preferable that an amount of an inner oil agent is small. However, the inorganic powder whose surface is treated by the fatty acid tends to have low dispersibility, as compared with an inorganic powder whose surface is treated by a silane coupling agent or silicone. Therefore, it is often the case that the powder whose surface is treated by the fatty acid is previously dispersed in the oil agent by using a bead mill or the like and is used as a dispersion. In a case where the dispersion obtained by dispersing the powder surface-treated by the fatty acid in the oil agent is used, since an oil content brought into a cosmetic from the dispersion is large, fresh feeling which is required of the O/W type emulsified cosmetic cannot be obtained.

In addition, when the conventional fatty acid-treated powder is blended in the inner oil phase of the O/W type emulsified cosmetic, fine particulate titanium oxide particles agglomerated due to insufficiency of dispersibility are forced out from emulsified particles, thereby causing problem in a temporal stability in that the emulsified particles unite at the forced fine particulate titanium oxide particles at starting points and others. Furthermore, also for the powder which is surface-treated by the silane coupling agent or the silicone, high dispersibility and temporal stability are required.

Therefore, an object of the present invention is provide a powder composition whose dispersibility is high.

SOLUTION TO PROBLEM

A powder composition according to the present invention includes a titanium oxide powder as powder to be treated, at least one hydrophobic treatment agent selected from the group consisting of a fatty acid, silicone, and a silane coupling agent, a surfactant, and an oil agent, a content of the surfactant is 0.1% or more and 50% or less of a weight of the powder to be treated, and a content of the oil agent is 0.1% or more and 50% or less of a weight of the powder to be treated.

Thus, a powder composition whose dispersibility is high can be provided.

DESCRIPTION OF EMBODIMENTS

Hereinafter, with reference to specific examples, a powder composition of the present invention and a cosmetic including the powder composition will be described in detail. Note that the present invention is not limited to the below-described embodiments and a variety of modifications can be made without departing from the scope of the technical idea of the present invention.

1. Manufacturing of powder composition

As one embodiment, a powder composition according to the present invention is manufactured by the below-described method.

1.1 Manufacturing by wet processing

In order to enhance dispersibility of a fine particulate titanium oxide and secure excellent ultraviolet ray protective effect upon blending the fine particulate titanium oxide into a cosmetic, transparency, and temporal stability, the present inventors focused attention on not only a content ratio of powder to be treated and a surfactant or an oil agent but also manufacturing steps. Particulate powder such as a titanium oxide has the property of easily agglomerating. In particular, agglomeration due to thermal energy upon drying is considered to be dominant. Although the agglomerated particles are loosened finally through a pulverizing step, there are many particles whose each secondary agglomeration diameter exceeds several $\mu$m. Therefore, the present inventors focused attention on how to suppress agglomeration upon drying. The present inventors have devoted themselves to earnest research. As a result, the present inventors succeeded in suppressing agglomeration upon drying and in enhancing wettability to an oil agent by substituting a surfactant and an oil agent for water which are present in peripheries of particulates when the particulates are in a highly dispersed state in water. Based on these findings by the present inventors, it is preferable that a method for manufacturing a powder composition according to the present invention is configured as described below.

It is preferable that the method for manufacturing a powder composition according to the present invention includes: an alkalinizing step in which alkali metal titanate is obtained by adding an alkali metal hydroxide to an aqueous dispersion of a hydrous titanium oxide; an acidizing step in which a titanium oxide including a rutile type crystals is obtained by adding hydrochloric acid to an aqueous dispersion of alkali metal titanate; and a mixing step in which at least a hydrophobic treatment agent of at least one selected from the group consisting of a fatty acid, silicone, and a silane coupling agent, a surfactant, and an oil agent are mixed to the titanium oxide obtained in the acidizing step. In the method for manufacturing a powder composition according to the present invention, it is further preferable that the mixing step is a mixing step in which a metal oxide or metal hydroxide, a hydrophobic treatment agent, a surfactant, and an oil agent is mixed to the titanium oxide obtained in the acidizing step. As the alkali metal hydroxide, lithium hydroxide, sodium hydroxide, or potassium hydroxide is used. It is preferable that the sodium hydroxide or the potassium hydroxide is used.

In the method for manufacturing a powder composition according to the present invention, it is preferable that the mixing step includes: a step in which a surfactant and an oil agent are added after mixing the titanium oxide and the hydrophobic treatment agent or after mixing the titanium oxide and the metal oxide or the metal hydroxide, and the hydrophobic treatment agent or a step in which the hydrophobic treatment agent, the surfactant, and the oil agent are added after mixing the titanium oxide and the metal oxide or the metal hydroxide.

Although the powder composition is manufactured specifically by the below-described steps as one example, the method for manufacturing the powder composition is not limited thereto.

(Step 1)

A titanium dioxide hydrate is obtained by inputting a sodium hydroxide aqueous solution to hydrous titanium oxide cake obtained by filtrating and cleaning hydrolysate generated by heating an aqueous solution of titanyl sulfate crystals while being agitated. A suspension of the obtained titanium dioxide hydrate is filtrated, the cake is sufficiently cleaned, water is added to the cake after cleaning to prepare slurry, and a concentration thereof is adjusted. Furthermore, hydrochloric acid is added while being agitated, water is added thereto to adjust a concentration, and the resultant is heated for aging, thereby preparing slurry containing rutile-type titanium dioxide crystals.

(Step 2)

Water-soluble aluminum salt is added to the obtained slurry, a sodium hydroxide aqueous solution is further added thereto, pH is adjusted, and the resultant is aged. After aging, the slurry is heated, a hydrophobic treatment agent is added thereto, and the resultant is aged.

(Step 3)

After aging, a sodium hydroxide aqueous solution or a sulfuric acid aqueous solution is added, thereby adjusting pH of the slurry.

(Step 4)

After adjusting the pH, a surfactant is added to the slurry and the resultant is aged. An addition amount of the surfactant is 0.1% or more and 50% or less of a weight of the powder to be treated.

(Step 5)

After aging, an oil agent is added to the slurry and the resultant is aged. An addition amount of the oil agent is 0.1% or more and 50% or less of the weight of the powder to be treated.

(Step 6)

After aging, a sodium hydroxide aqueous solution or a sulfuric acid aqueous solution is added thereto, pH of the slurry is adjusted, and after aging, the slurry is filtrated, cleaned, and dried. The obtained dried product is pulverized until a desired particle diameter is achieved, thereby allowing a powder composition to be manufactured.

1.2 A method for manufacturing by dry processing

The slurry obtained in Step 3 in the above-described wet processing is filtrated, cleaned, and dried and the dried product thereby obtained is pulverized until a desired particle diameter is achieved, thereby obtaining hydrophobized powder to be treated. The hydrophobized powder to be treated may be manufactured by other method, for example, a method in which the powder to be treated and the hydrophobic treatment agent are dry-mixed or a method in which the powder to be treated, the hydrophobic treatment agent, and a solvent are mixed, and thereafter, the solvent is removed by heating treatment or decompression treatment.

While the hydrophobized powder to be treated is agitated by a mixer and a surfactant and an oil agent are added thereto, and the obtained powder is pulverized until a desired particle diameter is achieved, thereby allowing a powder composition to be obtained. An addition amount of the surfactant is 0.1% or more and 50% or less of a weight of the powder to be treated and an addition amount of the oil agent is 0.1% or more and 50% or less of the weight of the powder to be treated.

2. Powder to be treated

Titanium oxide powder as the powder to be treated is manufactured by the heretofore known method. A crystal form of the powder to be treated is selected in accordance with an application. For example, it is preferable that the titanium oxide powder includes rutile type crystals. A particle diameter is selected in accordance with an application and for example, in a case in which the powder to be treated is blended to an emulsified cosmetic, it is preferable that an average primary particle diameter is 5 nm or more and 50 nm or less and it is more preferable that the average primary particle diameter is 8 nm or more and 30 nm. Here, the average primary particle diameter means a number average particle diameter of a Heywood diameter (projected area circle equivalent diameter) obtained by analyzing images of titanium oxide particles (number of particles: 100) shot by a transmission electron microscope (TEM) by image analysis type particle size measurement software.

3. Hydrophobic treatment agent

The hydrophobic treatment agent is at least one selected from the group consisting of a fatty acid, silicone, and a silane coupling agent and by mixing the hydrophobic treatment agent with the powder to be treated or by other method, the hydrophobic treatment agent is treated onto the powder to be treated, thereby coating at least one part or preferably, all part of a surface of the powder to be treated. As the fatty acid, it is preferable that stearic acid or its salt, lauric acid or its salt, myristic acid or its salt, palmitic acid or its salt, or isostearic acid or its salt is used and it is more preferable that the stearic acids or its salt or the isostearic acid or its salt is used. As the silicone, dimethicone, hydrogen dimethicone, methylphenyl polysiloxane, triethoxysilylethyl polydimethylsiloxyethyl hexyl dimethicone, trimethylsiloxysilicate, or the like can be used. As the silane coupling agent, methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, octyltriethoxysilane (triethoxycaprylylsilane), decyltrimethoxysilane, or the like can be used. It is preferable that a content of the hydrophobic treatment agent in the powder composition is 7% or more and 30% or less of the weight of the powder to be treated and it is more preferable that the content thereof is 10% or more and 30% or less. Note that in a case in which as the hydrophobic treatment agent, the salt of the fatty acid is used, a value of a content of the hydrophobic treatment agent in the powder composition is a value calculated by substituting hydrogen ions for counter ions contained in its salt (that is, a value in terms of the fatty acid).

4. Surfactant

The surfactant is mixed with the powder to be treated or is treated onto the powder to be treated by other method, thereby coating one part or preferably all of a surface of the powder to be treated. It is only required for the surfactant to physically attach to the surface of the powder to be treated and it is not needed for the surfactant to chemically bond to the surface of the powder to be treated. As the surfactant, from a point of view to enhance dispersibility to an oil phase of a cosmetic, it is preferable that a surfactant whose HLB is 10 or less is used and it is more preferable that a surfactant whose HLB is 8 or less is used. Specifically, used is PEG-5 phytosterol; PEG-10 hydrogenated castor oil; polyoxyethylene glyceryl isostearates; polyglyceryl-2 isostearate; polyglyceryl-4 isostearate; sorbitan olivate; glyceryl oleate; sorbitan oleate; polyglyceryl-2 oleate; polyglyceryl-4 oleate; oleth-2; polyglyceryl diisostearate; polyglyceryl-3 diisostearate; self-emulsifying propylene glycol monostearate; sucrose distearate; polyglyceryl polyricinoleate; glyceryl monooleate, lipophilic; glyceryl monostearate, lipophilic; PEG-2 stearate; PEG-25 stearate; propylene glycol stearate; propylene glycol stearate SE; glyceryl stearate; sorbitan stearate; polyglyceryl-2 stearate; polyglyceryl-4 stearate; steareth-2; steareth-3; sorbitan sesquiisostearate; sorbitan sesquioleate; sorbitan sesquistearate; ceteth-3; PEG-5 glyceryl triisostearate; PEG-10 glyceryl triisostearate; polyoxyethylene glyceryl triisostearate; sorbitan trioleate; sorbitan tristearate; sorbitan palmitate; glyceryl behenate; polyglyceryl pentaisostearate; polyglyceryl-10 pentaisostearate; polyglyceryl pentaoleate; polyglyceryl-10 pentaoleate; decaglyceryl pentastearate; polyglyceryl-10 pentastearate; polyoxyethylene isostearyl ether; polyoxyethylene oleyl ether; polyoxyethylene stearylether; polyoxyethylene phytosterol; polyoxyethylene behenyl ether; polyoxyethylene lauryl ether; sucrose polystearate; polyhydroxystearic acid; polyglyceryl-3 polyricinoleate; polyglyceryl-5 polyricinoleate; polyglyceryl-6 polyricinoleate; glyceryl monoisostearate; sorbitan monoisostearate; polyglyceryl monoisostearate; sorbitan monooleate; polyglyceryl monooleate; sorbitan monostearate; propyleneglycol monostearate; polyoxyethylene sorbitan monostearate; polyglyceryl monostearate; sorbitan monopalmitate; sorbitan monolaurate; sorbitan cocoate; sorbitan laurate; laureth-2; and diisostearyl malate is used, and in particular, it is preferable that polyhydroxystearic acid; diisostearyl malate; sorbitan sesquiisostearate; polyglyceryl-5 polyricinoleate; PEG-11 methyl ether dimethicone; PEG-10 dimethicone; PEG/PPG-20/22 butyl ether dimethicone; PEG-3 dimethicone; cetyl PEG/PPG-10/1 dimethicone; PEG-9 polydimethylsiloxyethyl dimethicone; lauryl PEG-9 polydimethylsiloxyethyl dimethicone; polyglyceryl-3 disiloxane dimethicone; polyglyceryl-3 polydimethylsiloxyethyl dimethicone; lauryl polyglyceryl-3 polydimethylsiloxyethyl dimethicone; acrylates/ethylhexyl acrylate/dimethicone methacrylate copolymer; cetyl diglyceryl tris (trimethylsiloxy) silylethyl dimethicone; lauryl PEG-10 tris (trimethylsiloxy) silylethyl dimethicone; or the like is used. A content of the surfactant in the powder composition is 0.1% or more and 50% or less of a weight of the powder to be treated, it is preferable that the content thereof is 0.1% or more and 30% or less, it is more preferable that the content thereof is 0.1% or more and 10% or less, it is further preferable that the content there of is 2% or more and 8% or less, and it is most preferable that the content there of is 4% or more and 6% or less.

5. Oil agent

The oil agent is mixed with the powder to be treated or is treated onto the powder to be treated by other method, thereby coating one part or preferably all of a surface of the powder to be treated. It is only required for the oil agent to physically attach to the surface of the powder to be treated and it is not needed for the surfactant to chemically bond to the surface of the powder to be treated. As the oil agent, it is preferable that isobutene; hydrogenated polyisobutene; isohexadecane; isodecane; isododecane; eicosane; isoeicosane; squalane; liquid paraffin; light liquid isoparaffin; heavy liquid isoparaffin; stearyl 2-ethylhexanoate; cetylic 2-ethylhexanoate; cetostearyl 2-ethylhexanoate; N-lauroyl sarcosine isopropyl; glyceryl (adipate/2-ethylhexanoate/stearate) oligoesters; diisobutyl adipate; diisopropyl adipate; C12-15 alkyl benzoate; C15-19 alkane; 2-hexyldecyl isostearate; isostearyl isostearate; isocetyl isostearate; isopropyl isostearate; ethyl isostearate; octyldodecyl isostearate; hardened castor oil isostearate; cholesteryl isostearate; hydrogenated castor oil isostearate; phytosteryl isostearate; hexyldecyl isostearate; pentaerythritol isostearate; 2-ethylhexyl isononanoate; isotridecyl isononanoate; isononyl isononanoate; ethylhexyl isononanoate; isotridecyl isopelargonate; octyl isopelargonate; stearyl ethylhexanoate; glyceryl ethylhexanoate/stearate/adipate; cetyl ethylhexanoate; cetearyl ethylhexanoate; octyl oxystearate; cholesteryl oxystearate; stearyl octanoate; cetyl octanoate; cetostearyl octanoate; ethyl oleate; acetyl triethyl citrate; acetyl tributyl citrate; tri(2-octyldodecyl) citrate; triethyl citrate; trioctyldodecyl citrate; tributyl citrate; a glycerin fatty acid ester adipic acid condensate; di-2-ethylhexyl succinate; diethylhexyl succinate; dioctyl succinate; neopentyl glycol diethylhexanoate; glyceryl diisostearate; neopentyl glycol diethylhexanoate; neopentyl glycol diethylhexanoate; propylene glycol dicaprylate; propylene glycol dicaprylate/dicaprate; propylene glycol dicaprylate/dicaprate; propylene glycol dicaprylate; propylene glycol dicaprate; neopentyl glycol dicaprate; propylene glycol dicaprate; diisopropyl dilinoleate; 2-ethylhexyl stearate; ethylhexyl stearate; butyl stearate; diisopropyl dimerate; hydrogenated castor oil dimer dilinoleate; C14-15 dialkyl carbonate; dicaprylyl carbonate; pentaerythrityl tetra(2-ethylhexanoate); pentaerythrityl tetraisostearate; pentaerythritol tetraisostearate; pentaerythrityl tetraethylhexanoate; pentaerythrityl tetraoctanoate; dipentaerythrityl tetrahydroxystearate/tetraisostearate; glyceryl tri(2-ethylhexanoate); trimethylolpropane tri(2-ethylhexanoate); triisostearin; glyceryl triisostearate; triethylhexanoin; trimethylolpropane triethylhexanoate; glyceryl trioctanoate; trimethylolpropane triethylhexanoate; tricaprylin; caprylic/capric triglyceride; caprylic/capric/myristic/stearic triglyceride; caprylic/capric triglyceride; caprylic/capric/myristic/stearic triglyceride; caprylic triglyceride; tribehenin; glyceryl tribehenate; octyldodecyl lactate; cetyl lactate; myristyl lactate; 2-octyldodecyl neopentanoate; octyldodecyl neopentanoate; 2-ethylhexyl palmitate; isopropyl palmitate; ethylhexyl palmitate; octyl palmitate; cetyl palmitate; 2-ethylhexyl hydroxystearate; ethylhexyl hydroxystearate; cholesteryl hydroxystearate; phytosteryl hydroxystearate; 2-octyldodecyl pivalate; dipentaerythrityl hexahydroxystearate; dipentaerythritol hexahydroxystearate; dipentaerythrityl hexahydroxystearate/hexastearate/hexarosinate; isostearyl myristate; octyldodecyl myristate; myristyl myristate; hexyl laurate; methylheptyl laurate; isopropyl lauroyl sarcosinate; or diisostearyl malate is used, and in particular, it is preferable that hydrogenated polyisobutene; C12-15 alkyl benzoate; squalane; C15-19 alkane; caprylic/capric triglyceride; triethylhexanoin; hexyl laurate; cyclopentasiloxane; dimethicone; trisiloxane; ethyl methicone; methyl trimethicone; diphenylsiloxy phenyl trimethicone; caprylyl methicone; or the like is used. A content of the oil agent in the powder composition is 0.1% or more and 50% or less of a weight of the powder to be treated, it is preferable that the content thereof is 0.1% or more and 30% or less, it is more preferable that the content thereof is 0.1% or more and 10% or less, it is further preferable that the content thereof is 2% or more and 8% or less, and it is most preferable that the content thereof is 4% or more and 6% or less.

6. Metal oxide or metal hydroxide

When the powder to be treated is manufactured, a metal oxide or a metal hydroxide may be added to the powder to be treated and mixed. Alternatively, a water-soluble metal salt may be added to water slurry of the powder to be treated and a sodium hydroxide aqueous solution or a sulfuric acid aqueous solution may be added thereto, thereby adjusting pH and conducting aging, whereby the metal oxide or the metal hydroxide may be mixed in the powder to be treated. As the metal oxide or the metal hydroxide, for example, an oxide or a hydroxide of each of aluminum, zinc, titanium, zirconium, silicon, and cerium is used and it is preferable that the oxide or the hydroxide of the aluminum is used. As effects of these metal oxides or metal hydroxides, mainly, suppression of photoactivity of the powder to be treated is cited.

7. Property of powder composition

The powder composition of the present invention may be of any property from in a powder state to in a paste state having no fluidity as a liquid. For example, the powder composition may be in a bean-curd leftover state.

8. Cosmetic

Since the powder composition of the present invention has extremely high dispersibility, when the powder composition thereof is blended in an inner oil phase of an O/W type emulsified cosmetic, there is little agglomeration and an emulsified cosmetic having high stability can be provided. The powder composition of the present invention exhibits ultraviolet ray protective effect also in a case in which the powder composition thereof is blended in an oil phase of a W/O type emulsified cosmetic. A cosmetic in which the powder composition of the present invention is blended is not limited. For example, as a raw material which imparts the ultraviolet ray protective effect thereto, only the powder composition of the present invention may be blended in a cosmetic or the powder composition of the present invention may be blended therein with other raw material having the ultraviolet ray protective effect (an titanium oxide, a zinc oxide, a cerium oxide, an iron oxide, an organic ultraviolet absorber, or the like) in combination. At this time, the raw material to be blended in combination, which has the ultraviolet ray protective effect, may be blended in any of a water phase and an oil phase.
As a method in a case in which the powder composition of the present invention is blended in the O/W type emulsified cosmetic, for example, a method in which the powder composition of the present invention is added to a mixture which is an oil phase raw material and while the resultant is being agitated, a mixture of a water phase raw material can be added is employed.

The present invention is summarized as follows.

(1) The powder composition according to the present invention includes: titanium oxide powder as the powder to be treated; a hydrophobic treatment agent of at least one selected from the group consisting of a fatty acid, silicone, and a silane coupling agent; a surfactant; and an oil agent, and a content of the surfactant is 0.1% or more and 50% or less of a weight of the powder to be treated and a content of the oil agent is 0.1% or more and 50% or less of the weight of the powder to be treated.

(2) In the (1) powder composition of the present invention, it is preferable that a content of the hydrophobic treatment agent is 7% or more and 30% or less of the weight of the powder to be treated.

(3) It is preferable that a total light transmittance of the (1) or (2) powder composition of the present invention at a wavelength of 300 nm is 10% or less and a value of (300 nm-transmittance)/(400 nm-transmittance) is 0.2 or less.

(4) The cosmetic according to the present invention includes the powder composition according to any of (1) to (3).

(5) The method for manufacturing a powder composition according to the present invention includes: the alkalinizing step in which the alkali metal hydroxide is added to the aqueous dispersion of the hydrous titanium oxide and alkali metal titanate is thereby obtained; the acidizing step in which the hydrochloric acid is added to the aqueous dispersion of the alkali metal titanate and the titanium oxide including the rutile type crystals is obtained; and the mixing step in which at least, at least one hydrophobic treatment agent selected from the group consisting of the fatty acid, the silicone, and the silane coupling agent, the surfactant, and the oil agent are added to the titanium oxide obtained in the acidizing step and mixed.

(6) In the (5) method of the present invention, it is preferable that after mixing the titanium oxide and the hydrophobic treatment agent, the mixing step includes adding the surfactant and the oil agent.

(7) In the (5) method of the present invention, it is preferable that the mixing step is a step in which at least the metal oxide or the metal hydroxide, the hydrophobic treatment agent, the surfactant, and the oil agent are mixed with the titanium oxide obtained in the acidizing step and mixed.

(8) In the (7) method of the present invention, it is preferable that the mixing step includes adding the surfactant and the oil agent after mixing the titanium oxide, the metal oxide or the metal hydroxide, and the hydrophobic treatment agent.

(9) In the (7) method of the present invention, it is preferable that the mixing step includes adding the hydrophobic treatment agent, the surfactant, and the oil agent after mixing the titanium oxide and the metal oxide or the metal hydroxide.

[Examples]

With reference to specific manufacturing examples and test results of the cosmetics using each of the powder compositions according to the present invention, further detailed description will be given.

<Manufacturing of powder compositions>

Example 1

(Step 1) Inputted to 35 kg of hydrous titanium oxide cake (a content of a titanium oxide: corresponding to 10 kg in terms of $TiO_2$) obtained by filtrating and cleaning hydrolysate generated by heating an aqueous solution of titanyl sulfate crystals (manufactured by TAYCA CORPORATION, TM crystal) was 40 kg of a 48%-sodium hydroxide aqueous solution while being agitated and the resultant was heated at a range of 95°C to 105°C and was agitated for 2 hours. Next, a suspension of this titanium dioxide hydrate was filtrated and the cake was sufficiently cleaned. Approximately 25 kg of water was added to the cake after cleaning and the cake was made into a slurry and a concentration thereof was adjusted to 220 g/L in terms of $TiO_2$. Furthermore, 14 kg of 35% hydrochloric acid was inputted thereto while being agitated and water was added, thereby adjusting the concentration to 160 g/L in terms of $TiO_2$. Inputted hereto was 14.0 kg of 35% hydrochloric acid and thermal aging was conducted at 95°C to 100°C for 2 hours. Solid particles in this slurry exhibited a crystal structure of a rutile-type titanium dioxide in X-ray diffraction and an average primary particle diameter (an average primary particle diameter of a Heywood diameter (projected area circle equivalent diameter) obtained by analyzing images of titanium oxide particles (number of particles: 100) shot by a transmission electron microscope (TEM) by image analysis type particle size measurement software (MOUNTECH Co., Ltd.: Mac-View)) was 15 nm.

(Step 2) A concentration of the obtained titanium oxide slurry was adjusted to 70 g/L. Added to 20 L of this slurry (1.4 kg in terms of $TiO_2$) was 1,133 mL of a polyaluminum chloride aqueous solution, which was 123.6 g/L in terms of $Al_2O_3$, (10 weight% in terms of $Al_2O_3$ based on a titanium dioxide) while being agitated. A sodium hydroxide aqueous solution was added thereto, a pH value was adjusted to 6.0, and aging was conducted for 30 minutes. After heating the slurry to 85°C, 248 g of sodium stearate (16.5 weight% in terms of a fatty acid with respect to the titanium dioxide) was added. At this time, a pH value gradually increased and 10 minutes later, reached pH 7.0. This slurry was aged for one hour.

(Step 3) After aging, the pH value of the slurry was adjusted to 7.5 by adding a sodium hydroxide aqueous solution or a sulfuric acid aqueous solution.

(Step 4) Added was 70 g of polyhydroxystearic acid (manufactured by The Nisshin OilliO Group, Ltd./trade name: SALACOS HS-6C) (5 weight% in terms of an active component with respect to the titanium dioxide) and aging was conducted for 15 minutes.

(Step 5) After aging, 70 g of hexyl laurate (manufactured by KOKYU ALCOHOL KOGYO CO., LTD./trade name: KAK HL) (5 weight% in terms of an active component with respect to titanium dioxide) was added and aging was conducted for 30 minutes.

(Step 6) After aging, a pH value of the slurry was adjusted to 5.5 by adding a sodium hydroxide aqueous solution or a sulfuric acid aqueous solution, thereafter, aging was conducted for 10 minutes, the slurry was filtrated, cleaned, and dried at 85°C for 15 hours, and a dried product was pulverized by an EC atomizer, thereby manufacturing a powder composition in Example 1.

Example 2

A powder composition was manufactured in the same manner as in Example 1 except that the sodium stearate in Step 2 was changed to sodium laurate.

Example 3

A powder composition was manufactured in the same manner as in Example 1 except that the sodium stearate in Step 2 was changed to sodium myristate.

Example 4

A powder composition was manufactured in the same manner as in Example 1 except that the sodium stearate in Step 2 was changed to sodium palmitate.

Example 5

A powder composition was manufactured in the same manner as in Example 1 except that the sodium stearate in Step 2 was changed to isostearic acid.

Example 6

A powder composition was manufactured in the same manner as in Example 1 except that an addition amount of the polyhydroxystearic acid in Step 4 was changed to 1.4 g (0.1 weight% in terms of an active component with respect to the titanium dioxide).

Example 7

A powder composition was manufactured in the same manner as in Example 1 except that an addition amount of the polyhydroxystearic acid in Step 4 was changed to 140 g (10 weight% in terms of an active component with respect to the titanium dioxide).

Example 8

A powder composition was manufactured in the same manner as in Example 1 except that an addition amount of the hexyl laurate in Step 5 was changed to 1.4 g (0.1 weight% in terms of an active component with respect to the titanium dioxide).

Example 9

A powder composition was manufactured in the same manner as in Example 1 except that an addition amount of the hexyl laurate in Step 5 was changed to 140 g (10 weight% in terms of an active component with respect to the titanium dioxide).

Example 10

A powder composition was manufactured in the same manner as in Example 1 except that an addition amount of the sodium stearate in Step 2 was changed to 155 g (10.3 weight% in terms of the fatty acid with respect to the titanium dioxide).

Example 11

A powder composition was manufactured in the same manner as in Example 1 except that an addition amount of the sodium stearate in Step 2 was changed to 440 g (29.2 weight% in terms of the fatty acid with respect to the titanium dioxide).

Example 12

A powder composition was manufactured in the same manner as in Example 1 except that the polyhydroxystearic acid in Step 4 was changed to diisostearyl malate.

Example 13

A powder composition was manufactured in the same manner as in Example 1 except that the polyhydroxystearic acid in Step 4 was changed to sorbitan sesquiisostearate.

Example 14

A powder composition was manufactured in the same manner as in Example 1 except that the polyhydroxystearic acid in Step 4 was changed to polyglyceryl-5 polyricinoleate.

Example 15

A powder composition was manufactured in the same manner as in Example 1 except that the hexyl laurate in Step 5 was changed to hydrogenated polyisobutene.

Example 16

A powder composition was manufactured in the same manner as in Example 1 except that the hexyl laurate in Step 5 was changed to isododecane.

Example 17

A powder composition was manufactured in the same manner as in Example 1 except that the hexyl laurate in Step 5 was changed to C15-19 alkane.

Example 18

A powder composition was manufactured in the same manner as in Example 1 except that the hexyl laurate in Step 5 was changed to squalane.

Example 19

A powder composition was manufactured in the same manner as in Example 1 except that the hexyl laurate in Step 5 was changed to C12-15 alkyl benzoate.

Example 20

A powder composition was manufactured in the same manner as in Example 1 except that the hexyl laurate in Step 5 was changed to caprylic/capric triglyceride.

Example 21

A powder composition was manufactured in the same manner as in Example 1 except that the hexyl laurate in Step 5 was changed to triethylhexanoin.

Example 22

Inputted to 35 kg of hydrous titanium oxide cake (a content of a titanium oxide: corresponding to 10 kg in terms of $TiO_2$) obtained by filtrating and cleaning hydrolysate generated by heating an aqueous solution of titanyl sulfate crystals (manufactured by TAYCA CORPORATION, TM crystal) was 100 kg of a 48%-sodium hydroxide aqueous solution while being agitated and the resultant was heated at a range of 95°C to 105°C and was agitated for 2 hours. Next, a suspension of a this titanium dioxide hydrate was filtrated and the cake was sufficiently cleaned. Approximately 25 kg of water was added to the cake after cleaning and the cake was made into a slurry and a concentration thereof was adjusted to 220 g/L in terms of $TiO_2$. Furthermore, 1.7 kg of 35% hydrochloric acid was inputted thereto while being agitated and water was added, thereby adjusting the concentration to 160 g/L in terms of $TiO_2$. After 250 g of sodium sulfate anhydride was inputted hereto, 14.0 kg of 35% hydrochloric acid was inputted thereto and thermal aging at 95°C to 100°C was conducted for 2 hours. Solid particles in this slurry exhibited a crystal structure of a rutile-type titanium dioxide in X-ray diffraction and an average primary particle diameter was 8 nm.

A concentration of the obtained titanium oxide slurry was adjusted to 70 g/L. Added to 20 L of this slurry (1.4 kg in terms of $TiO_2$) was 1,472 mL of a polyaluminum chloride aqueous solution, which was 123.6 g/L in terms of $Al_2O_3$, (13 weight% in terms of $Al_2O_3$ based on a titanium dioxide) while being agitated. A sodium hydroxide aqueous solution was added thereto, a pH value was adjusted to 6.0, and aging was conducted for 30 minutes. After heating the slurry to 85°C, 440 g of sodium stearate (29.2 weight% in terms of a fatty acid with respect to the titanium dioxide) was added. At this time, a pH value gradually increased and 10 minutes later, reached pH 7.0. This slurry was aged for one hour.

After aging, the pH value of the slurry was adjusted to 7.5 by adding a sodium hydroxide aqueous solution or a sulfuric acid aqueous solution and 98 g of polyhydroxystearic acid (7 weight% in terms of an active component with respect to the titanium dioxide) was added and aging was conducted for 15 minutes.

After aging, 98 g of hexyl laurate (7 weight% in terms of an active component with respect to the titanium dioxide) was added and aging was conducted for 30 minutes.

After aging, a pH value of the slurry was adjusted to 5.5 by adding a sodium hydroxide aqueous solution or a sulfuric acid aqueous solution, thereafter, aging was conducted for 10 minutes, the slurry was filtrated, cleaned, and dried at 85°C for 15 hours, and a dried product was pulverized by an EC atomizer, thereby manufacturing a powder composition.

Example 23

Inputted to a mixer were 500 g of commercially available titanium oxide powder (manufactured by TAYCA CORPORATION: "MT-100Z", an average primary particle diameter: 15 nm, surface treatment agent: aluminum (10 weight% in terms of $Al_2O_3$ with respect to the titanium dioxide) and stearic acid (16.5 weight% with respect to the titanium dioxide)), 20 g of polyhydroxystearic acid (5 weight% in terms of an active component with respect to the titanium dioxide) was added while being agitated, and agitation was conducted for 10 minutes. Furthermore, while agitation was continued, 20 g of hexyl laurate (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 24

Inputted to 35 kg of hydrous titanium oxide cake (a content of a titanium oxide: corresponding to 10 kg in terms of $TiO_2$) obtained by filtrating and cleaning hydrolysate generated by heating an aqueous solution of titanyl sulfate crystals (manufactured by TAYCA CORPORATION, TM crystal) was 100 kg of a 48%-sodium hydroxide aqueous solution while being agitated and the resultant was heated at a range of 95°C to 105°C and was agitated for 2 hours. Next, a suspension of this titanium dioxide hydrate was filtrated and the cake was sufficiently cleaned. Approximately 25 kg of water was added to the cake after cleaning and the cake was made into a slurry and a concentration thereof was adjusted to 220 g/L in terms of $TiO_2$. Furthermore, 1.7 kg of 35% hydrochloric acid was inputted thereto while being agitated and water was added, thereby adjusting the concentration to 160 g/L in terms of $TiO_2$. After 250 g sodium sulfate anhydride was inputted hereto, 14.0 kg of 35% hydrochloric acid was inputted thereto and thermal aging at 95°C to 100°C was conducted for 2 hours. Solid particles in this slurry exhibited a crystal structure of a rutile-type titanium dioxide in X-ray diffraction.

A concentration of the obtained titanium oxide slurry was adjusted to 70 g/L. Added to 20 L of this slurry (1.4 kg in terms of $TiO_2$) was ammonia water, thereby adjusting a pH value to 6.0, the slurry was heated to 60°C, aging was conducted, thereafter, the pH value was adjusted to 6.0 by the ammonia water again, and the slurry was filtrated and cleaned, thereby obtaining cleaned cake. This cake was dispersed in water again and was heated to 60°C, the ammonia water was added, thereby adjusting the pH value to 7.5, and aging was conducted. After aging, the pH value was adjusted to 7.5 by the ammonia water and the slurry was filtrated and cleaned, thereby obtaining cleaned cake. The cleaned cake was putted into a drier (150°C) and moisture was thereby removed, thereby obtaining titanium oxide powder (dried product).

The titanium oxide powder (dried product) was burned at 480°C for 120 minutes and was pulverized, thereby obtaining titanium oxide powder (burned product). An average primary particle diameter of the titanium oxide powder (burned product) was 30 nm. Water was added to the obtained titanium oxide powder (burned product), thereby adjusting a concentration to 10 g/L. Heated to 85°C was 10 L of the above-mentioned titanium oxide slurry (1.0 kg in terms of $TiO_2$) and 115 g of sodium stearate (10.7 weight% in terms of the fatty acid with respect to the titanium dioxide) was added. At this time, a pH value gradually increased and 10 minutes later, reached pH 7.0. This slurry was aged for one hour. The pH value after one-hour aging was 9.0. After aging, the pH value of the slurry was adjusted to pH 6.7 by adding a sodium hydroxide aqueous solution or a sulfuric acid aqueous solution, thereafter, aging was conducted for 30 minutes, the slurry was filtrated and cleaned, drying was conducted at 105°C for 15 hours, and the dried product was pulverized by an EC atomizer. Inputted to a mixer was 500 g of the titanium oxide powder obtained as described above as in Example 23 and treatment was conducted by using polyhydroxystearic acid and hexyl laurate in the same conditions. The titanium oxide powder obtained as described above was pulverized by an EC atomizer, thereby manufacturing a powder composition.

Example 25

A powder composition was manufactured in the same manner as in Example 1 except that the polyhydroxystearic acid in Step 5 was changed to sorbitan sesquiisostearate and the hexyl laurate was changed to C15-19 alkane.

Example 26

A powder composition was manufactured in the same manner as in Example 1 except that the polyhydroxystearic acid in Step 5 was changed to sorbitan sesquiisostearate and the hexyl laurate was changed to C12-15 alkyl benzoate.

Example 27

A powder composition was manufactured in the same manner as in Example 1 except that the polyhydroxystearic acid in Step 5 was changed to polyglyceryl-10 isostearate/succinate.

Example 28

A powder composition was manufactured in the same manner as in Example 1 except that the polyhydroxystearic acid in Step 5 was changed to polyglyceryl-5 polyricinoleate and the hexyl laurate was changed to C12-15 alkyl benzoate.

Example 29

A powder composition was manufactured in the same manner as in Example 1 except that the polyhydroxystearic acid in Step 5 was changed to polyglyceryl-5 polyricinoleate and the hexyl laurate was changed to C15-19 alkane.

Example 30

Inputted to a mixer were 500 g of commercially available titanium oxide powder (manufactured by TAYCA CORPORATION: "MT-100Z", an average primary particle diameter: 15 nm, a surface treatment agent: aluminum (10 weight% in terms of $Al_2O_3$ with respect to the titanium dioxide) and stearic acid (16.5 weight% with respect to the titanium dioxide)), 20 g of sorbitan sesquiisostearate (5 weight% in terms of an active component with respect to the titanium dioxide) was added while being agitated and agitation was conducted for 10 minutes. Furthermore, while agitation was continued and 20 g of C12-15 alkyl benzoate (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 31

Inputted to a mixer were 500 g of commercially available titanium oxide powder (manufactured by TAYCA CORPORATION: "MT-100Z", an average primary particle diameter: 15 nm, a surface treatment agent: aluminum (10 weight% in terms of $Al_2O_3$ with respect to the titanium dioxide) and stearic acid (16.5 weight% with respect to the titanium dioxide)), 20 g of sorbitan sesquiisostearate (5 weight% in terms of an active component with respect to the titanium dioxide) was added while being agitated and agitation was conducted for 10 minutes. Furthermore, while agitation was continued, 20 g of C15-19 alkane (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 32

Inputted to a mixer were 500 g of commercially available titanium oxide powder (manufactured by TAYCA CORPORATION: "MT-05", an average primary particle diameter: 10 nm, a surface treatment agent: silicon (5 weight% in terms of $SiO_2$ with respect to the titanium dioxide) and aluminum (10 weight% in terms of $Al_2O_3$ with respect to the titanium dioxide)), and while being agitated, 65 g of dimethicone (15 weight% in terms of an active component with respect to the titanium dioxide) was added and agitation was conducted for 15 minutes. Furthermore, 22 g of PEG-9 polydimethylsiloxyethyl dimethicone (5 weight% in terms of an active component with respect to the titanium dioxide) was added and agitation was conducted for 10 minutes. Furthermore, while agitation was continued, 22 g of dimethicone (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 33

Inputted to a mixer were 500 g of commercially available titanium oxide powder (manufactured by TAYCA CORPORATION: "MT-05", an average primary particle diameter: 10 nm, a surface treatment agent: silicon (5 weight% in terms of $SiO_2$ with respect to the titanium dioxide) and aluminum (10 weight% in terms of $Al_2O_3$ with respect to the titanium dioxide)), and while being agitated, 65 g of dimethicone (15 weight% in terms of an active component with respect to the titanium dioxide) was added and agitation was conducted for 15 minutes. Furthermore, 22 g of polyglyceryl-3 polydimethylsiloxyethyl dimethicone (5 weight% in terms of an active component with respect to the titanium dioxide) was added and agitation was conducted for 10 minutes. Furthermore, while agitation was continued, 22 g of dimethicone (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 34

Inputted to a mixer were 500 g of commercially available titanium oxide powder (manufactured by TAYCA CORPORATION: "MT-05", an average primary particle diameter: 10 nm, a surface treatment agent: silicon (5 weight% in terms of $SiO_2$ with respect to the titanium dioxide) and aluminum (10 weight% in terms of $Al_2O_3$ with respect to the titanium dioxide)) and while being agitated, 96 g of triethoxycaprylylsilane (22 weight% in terms of an active component with respect to the titanium dioxide) was added and agitation was conducted for 15 minutes. Furthermore, 22 g of sorbitan sesquiisostearate (5 weight% in terms of an active component with respect to the titanium dioxide) was added and agitation was conducted for 10 minutes. Furthermore, while agitation was continued, 22 g of C12-15 alkyl benzoate (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 35

Inputted to a mixer was 500 g of the titanium oxide powder (burned product) obtained in Example 24, 34 g of dimethicone (6.8 weight% in terms of an active component with respect to the titanium dioxide) was added, and agitation was conducted for 15 minutes. Furthermore, 25 g of PEG-9 polydimethylsiloxyethyl dimethicone (5 weight% in terms of an active component with respect to the titanium dioxide) was added and agitation was conducted for 10 minutes. Furthermore, while agitation was continued, 25 g of dimethicone (5 weight% in terms of an active component with respect to the titanium dioxide) was added and agitation was conducted for 15 minutes. The titanium oxide powder obtained as described above was pulverized by an EC atomizer, thereby manufacturing a powder composition.

Example 36

A concentration of the titanium oxide slurry (an average primary particle diameter: 8 nm) obtained in Example 22 was adjusted to 70 g/L. Added to 20 L of this slurry (1.4 kg in terms of $TiO_2$) was 1,472 mL of a polyaluminum chloride aqueous solution, which was 123.6 g/L in terms of $Al_2O_3$, (13 weight% in terms of $Al_2O_3$ based on a titanium dioxide) while being agitated. A sodium hydroxide aqueous solution was added thereto, a pH value was adjusted to 6.0, and aging was conducted for 30 minutes. After heating the slurry to 85°C, 440 g of sodium stearate (29.2 weight% in terms of a fatty acid with respect to the titanium dioxide) was added. At this time, a pH value gradually increased and 10 minutes later, reached pH 7.0. This slurry was aged for one hour. After aging, the pH value of the slurry was adjusted to 7.5 by adding a sodium hydroxide aqueous solution or a sulfuric acid aqueous solution, 700 g of sorbitan sesquiisostearate (50 weight% in terms of an active component with respect to the titanium dioxide) was added, and aging was conducted for 15 minutes. After aging, 70 g of C12-15 alkyl benzoate (5 weight% in terms of an active component with respect to the titanium dioxide) was added and aging was conducted for 30 minutes. After aging the pH value of the slurry was adjusted to 5.5 by adding the sodium hydroxide aqueous solution or the sulfuric acid aqueous solution, thereafter, aging was conducted for 10 minutes, the slurry was filtrated, cleaned, and dried at 85°C for 15 hours, and the dried product was pulverized by an EC atomizer, thereby manufacturing a powder composition.

Example 37

A concentration of the titanium oxide slurry (an average primary particle diameter: 8 nm) obtained in Example 22 was adjusted to 70 g/L. Added to 20 L of this slurry (1.4 kg in terms of $TiO_2$) was 1,472 mL of a polyaluminum chloride aqueous solution, which was 123.6 g/L in terms of $Al_2O_3$, (13 weight% in terms of $Al_2O_3$ based on a titanium dioxide) while being agitated. A sodium hydroxide aqueous solution was added thereto, a pH value was adjusted to 6.0, and aging was conducted for 30 minutes. After heating the slurry to 85°C, 440 g of sodium stearate (29.2 weight% in terms of a fatty acid with respect to the titanium dioxide) was added. At this time, a pH value gradually increased and 10 minutes later, reached pH 7.0. This slurry was aged for one hour. After aging, the pH value of the slurry was adjusted to 7.5 by adding a sodium hydroxide aqueous solution or a sulfuric acid aqueous solution, 70 g of sorbitan sesquiisostearate (5 weight% in terms of an active component with respect to the titanium dioxide) was added, and aging was conducted for 15 minutes. After aging, 700 g of C12-15 alkyl benzoate (50 weight% in terms of an active component with respect to the titanium dioxide) was added and aging was conducted for 30 minutes. After aging the pH value of the slurry was adjusted to 5.5 by adding the sodium hydroxide aqueous solution or the sulfuric acid aqueous solution, thereafter, aging was conducted for 10 minutes, the slurry was filtrated, cleaned, and dried at 85°C for 15 hours, and the dried product was pulverized by an EC atomizer, thereby manufacturing a powder composition.

Comparative Example 1

A powder composition was manufactured in the same manner as in Example 1 except that Step 4 and Step 5 were omitted.

Comparative Example 2

A powder composition was manufactured in the same manner as in Example 1 except that Step 5 was omitted.

Comparative Example 3

A powder composition was manufactured in the same manner as in Example 1 except that the addition amount of the polyhydroxystearic acid in Step 4 was changed to 140 g (10 weight% in terms of an active component with respect to the titanium dioxide) and Step 5 was omitted.

Comparative Example 4

A powder composition was manufactured in the same manner as in Example 1 except that Step 4 was omitted and the addition amount of the hexyl laurate in Step 5 was changed to 210 g (15 weight% in terms of an active component with respect to the titanium dioxide).

Comparative Example 5

A powder composition was manufactured in the same manner as in Example 1 except that the addition amount of the polyhydroxystearic acid in Step 4 was changed to 770 g (55 weight% in terms of an active component with respect to the titanium dioxide).

Comparative Example 6

A powder composition was manufactured in the same manner as in Example 1 except that the addition amount of the hexyl laurate in Step 5 was changed to 770 g (55 weight% in terms of an active component with respect to the titanium dioxide).

Comparative Example 7

Inputted to a mixer were 500 g of commercially available titanium oxide powder (manufactured by TAYCA CORPORATION: "MT-05", an average primary particle diameter: 10 nm, a surface treatment agent: silicon (5 weight% in terms of $SiO_2$ with respect to the titanium dioxide) and aluminum (10 weight% in terms of $Al_2O_3$ with respect to the titanium dioxide)), and while being agitated, 65 g of dimethicone (15 weight% in terms of an active component with respect to the titanium dioxide) was added and agitation was conducted for 15 minutes. The titanium oxide powder obtained as described above was pulverized by an EC atomizer, thereby manufacturing a powder composition.

Comparative Example 8

Inputted to a mixer were 500 g of commercially available titanium oxide powder (manufactured by TAYCA CORPORATION: "MT-05", an average primary particle diameter: 10 nm, a surface treatment agent: silicon (5 weight% in terms of $SiO_2$ with respect to the titanium dioxide) and aluminum (10 weight% in terms of $Al_2O_3$ with respect to the titanium dioxide)), and while being agitated, 96 g of triethoxycaprylylsilane (22 weight% in terms of an active component with respect to the titanium dioxide) was added and agitation was conducted for 15 minutes. The titanium oxide powder obtained as described above was pulverized by an EC atomizer, thereby manufacturing a powder composition.

<Dispersibility test>

Dispersibility of the manufactured powder compositions was evaluated as follows.

(1) Measured was 27.9 g of a medium, which was put into a 100 mL-cup formed of polypropylene and having a size of 50 mm-caliber $\times$ 43 mm-lower diameter $\times$ 76 mm-height. Here, for each of powder compositions in Example 32, Example 33, Example 35, and Comparative Example 7, as the medium, dimethicone (manufactured by Shin-Etsu Chemical Co., Ltd.: KF-96L-1.5cs) was used, and for each of powder compositions in other Examples and Comparative Examples, as the medium, isododecane (manufactured by Maruzen Petrochemical: Marukasol R) was used.
(2) Measured was 2.1 g of each of the powder compositions, which was added to the medium measured in (1).
(3) This was agitated at an agitation speed of 500 rpm for 10 minutes by using a high-speed emulsifying and dispersing machine (manufactured by PRIMIX Corporation, "HOMOGENIZING DISPER Model 2.5", dispersion blades each having $\varphi$30 mm).

Each of the dispersion liquids prepared as descried above was applied to polypropylene film (manufactured by RM TOHCELLO CO., LTD.: plain OPP sheet #40) by using an automatic bar coater with a wire bar (No.6) attached, thereby forming coated film (film thickness: 10 $\mu$m).
Total light transmittance of this coated film was measured by a spectrophotometer (manufactured by Hitachi High-Tech Corporation "U-4100", using an integrating sphere) under the following conditions.

Scanning speed: 300 nm/minute
Sampling interval: 2 nm
Measurement wavelength: 250 nm to 700 nm

Transmittance $T_{t300}$ at a wavelength of 300 nm and transmittance $T_{t400}$ at a wavelength of 400 nm were read from the obtained transmittance curve and a ratio was calculated by the following equation.

$$\text{Ratio} = T_{t300}/T_{t400}$$

Results are shown in Table 1. Evaluation was made such that when total light transmittance at the wavelength of 300 nm was 10% or less and a value of (300 nm-transmittance)/(400 nm-transmittance) was 0.2 or less, "Good" was given and when at least one of the above-mentioned conditions was not satisfied, "Bad" was given.

[Table 1]

| Powder composition | Processing method | Powder to be treated | | Metal oxide or metal hydroxide | | Hydrophobic treatment agent | | Surfactant | | Oil agent | | Transmittance | | | Evaluation |
|---|---|---|---|---|---|---|---|---|---|---|---|---|---|---|---|
| | | Kind | Particle diameter [nm] | Kind | Content | Kind | Content | Kind | Content | Kind | Content | 400 nm | 300 nm | 300/400 | |
| Example 1 | Wet | Titanium oxide | 15 | Aluminum | 10% | Stearic acid | 16.5% | Polyhydroxystearic acid | 5% | Hexyl laurate | 5% | 81.6% | 4.4% | *0.05* | Good |
| Example 2 | Wet | Titanium oxide | 15 | Aluminum | 10% | Lauric acid | 16.5% | Polyhydroxystearic acid | 5% | Hexyl laurate | 5% | 90.2% | 8.6% | 0.10 | Good |
| Example 3 | Wet | Titanium oxide | 15 | Aluminum | 10% | Myristic acid | 16.5% | Polyhydroxystearic acid | 5% | Hexyl laurate | 5% | 85.3% | 9.1% | 0.11 | Good |
| Example 4 | Wet | Titanium oxide | 15 | Aluminum | 10% | Palmitic acid | 16.5% | Polyhydroxystearic acid | 5% | Hexyl laurate | 5% | 86.2% | 8.5% | 0.10 | Good |
| Example 5 | Wet | Titanium oxide | 15 | Aluminum | 10% | Isostearic acid | 16.5% | Polyhydroxystearic acid | 5% | Hexyl laurate | 5% | 89.5% | 9.5% | 0.11 | Good |
| Example 6 | Wet | Titanium oxide | 15 | Aluminum | 10% | Stearic acid | 16.5% | Polyhydroxystearic acid | 0.1% | Hexyl laurate | 5% | 82.5% | 9.8% | 0.12 | Good |
| Example 7 | Wet | Titanium oxide | 15 | Aluminum | 10% | Stearic acid | 16.5% | Polyhydroxystearic acid | 10% | Hexyl laurate | 5% | 85.3% | 8.6% | 0.10 | Good |
| Example 8 | Wet | Titanium oxide | 15 | Aluminum | 10% | Stearic acid | 16.5% | Polyhydroxystearic acid | 5% | Hexyl laurate | 0.1% | 85.4% | 8.9% | 0.10 | Good |
| Example 9 | Wet | Titanium oxide | 15 | Aluminum | 10% | Stearic acid | 16.5% | Polyhydroxystearic acid | 5% | Hexyl laurate | 10% | 86.9% | 6.9% | 0.08 | Good |
| Example 10 | Wet | Titanium oxide | 15 | Aluminum | 10% | Stearic acid | 10.3% | Polyhydroxystearic acid | 5% | Hexyl laurate | 5% | 88.6% | 9.8% | 0.11 | Good |
| Example 11 | Wet | Titanium oxide | 15 | Aluminum | 10% | Stearic acid | 29.2% | Polyhydroxystearic acid | 5% | Hexyl laurate | 5% | 75.6% | 2.1% | 0.03 | Good |
| Example 12 | Wet | Titanium oxide | 15 | Aluminum | 10% | Stearic acid | 16.5% | Diisostearyl malate | 5% | Hexyl laurate | 5% | 82.6% | 6.5% | 0.08 | Good |
| Example 13 | Wet | Titanium oxide | 15 | Aluminum | 10% | Stearic acid | 16.5% | Sorbitan sesquiisostearate | 5% | Hexyl laurate | 5% | 84.6% | 6.4% | 0.08 | Good |
| Example 14 | Wet | Titanium oxide | 15 | Aluminum | 10% | Stearic acid | 16.5% | Polyglyceryl-5 polyricinoleate | 5% | Hexyl laurate | 5% | 86.6% | 5.6% | 0.06 | Good |

| Powder composition | Processing method | Powder to be treated | | Metal oxide or metal hydroxide | | Hydrophobic treatment agent | | Surfactant | | Oil agent | | Transmittance | | | Evaluation |
|---|---|---|---|---|---|---|---|---|---|---|---|---|---|---|---|
| | | Kind | Particle diameter [nm] | Kind | Content | Kind | Content | Kind | Content | Kind | Content | 400 nm | 300 nm | 300/400 | |
| Example 15 | Wet | Titanium oxide | 15 | Aluminum | 10% | Stearic acid | 16.5% | Polyhydroxystearic acid | 5% | Hydrogenated polyisobutene | 5% | 85.4% | 9.6% | 0.11 | Good |
| Example 16 | Wet | Titanium oxide | 15 | Aluminum | 10% | Stearic acid | 16.5% | Polyhydroxystearic acid | 5% | Isododecane | 5% | 76.5% | 8.5% | 0.11 | Good |
| Example 17 | Wet | Titanium oxide | 15 | Aluminum | 10% | Stearic acid | 16.5% | Polyhydroxystearic acid | 5% | C15-19 alkane | 5% | 78.5% | 7.9% | 0.10 | Good |
| Example 18 | Wet | Titanium oxide | 15 | Aluminum | 10% | Stearic acid | 16.5% | Polyhydroxystearic acid | 5% | Squalane | 5% | 76.5% | 9.5% | 0.12 | Good |
| Example 19 | Wet | Titanium oxide | 15 | Aluminum | 10% | Stearic acid | 16.5% | Polyhydroxystearic acid | 5% | C12-15 alkyl benzoate | 5% | 74.5% | 8.6% | 0.12 | Good |
| Example 20 | Wet | Titanium oxide | 15 | Aluminum | 10% | Stearic acid | 16.5% | Polyhydroxystearic acid | 5% | Caprylic/Capric Triglyceride | 5% | 79.8% | 6.5% | 0.08 | Good |
| Example 21 | Wet | Titanium oxide | 15 | Aluminum | 10% | Stearic acid | 16.5% | Polyhydroxystearic acid | 5% | Triethylhexanoin | 5% | 80.2% | 8.4% | 0.10 | Good |
| Example 22 | Wet | Titanium oxide | 8 | Aluminum | 13% | Stearic acid | 29.2% | Polyhydroxystearic acid | 7% | Hexyl laurate | 7% | 90.4% | 2.2% | 0.02 | Good |
| Example 23 | Dry | Titanium oxide | 15 | Aluminum | 10% | Stearic acid | 16.5% | Polyhydroxystearic acid | 5% | Hexyl laurate | 5% | 88.7% | 5.5% | 0.06 | Good |
| Example 24 | Dry | Titanium oxide | 30 | - | - | Stearic acid | 10.7% | Polyhydroxystearic acid | 5% | Hexyl laurate | 5% | 75.4% | 3.5% | 0.05 | Good |
| Example 25 | Wet | Titanium oxide | 15 | Aluminum | 10% | Stearic acid | 16.5% | Sorbitan sesquiisostearate | 5% | C15-19 alkane | 5% | 80.3% | 8.6% | 0.11 | Good |
| Example 26 | Wet | Titanium oxide | 15 | Aluminum | 10% | Stearic acid | 16.5% | Sorbitan sesquiisostearate | 5% | C12-15 alkyl benzoate | 5% | 79.5% | 4.0% | 0.05 | Good |
| Example 27 | Wet | Titanium oxide | 15 | Aluminum | 10% | Stearic acid | 16.5% | Polyglyceryl-10 Isostearate/Succinate | 5% | Hexyl laurate | 5% | 75.0% | 1.5% | 0.02 | Good |
| Example 28 | Wet | Titanium oxide | 15 | Aluminum | 10% | Stearic acid | 16.5% | Polyglyceryl-5 polyricinoleate | 5% | C12-15 alkyl benzoate | 5% | 77.8% | 5.0% | 0.06 | Good |

(continued)

| Powder composition | Processing method | Powder to be treated | | Metal oxide or metal hydroxide | | Hydrophobic treatment agent | | Surfactant | | Oil agent | | Transmittance | | | Evaluation |
|---|---|---|---|---|---|---|---|---|---|---|---|---|---|---|---|
| | | Kind | Particle diameter [nm] | Kind | Content | Kind | Content | Kind | Content | Kind | Content | 400 nm | 300 nm | 300/400 | |
| Example 29 | Wet | Titanium oxide | 15 | Aluminum | 10% | Stearic acid | 16.5% | Polyglyceryl-5 polyricinoleate | 5% | C15-19 alkane | 5% | 79.5% | 6.5% | 0.08 | Good |
| Example 30 | Dry | Titanium oxide | 15 | Aluminum | 10% | Stearic acid | 16.5% | Sorbitan sesquiisostearate | 5% | C12-15 alkyl benzoate | 5% | 80.5% | 8.0% | 0.10 | Good |
| Example 31 | Dry | Titanium oxide | 15 | Aluminum | 10% | Stearic acid | 16.5% | Sorbitan sesquiisostearate | 5% | C15-19 alkane | 5% | 80.3% | 8.6% | 0.11 | Good |
| Example 32 | Dry | Titanium oxide | 10 | Silicon aluminum | 5% 10% | Dimethicone | 15.0% | PEG-9 polydimethylsiloxyethyl dimethicone | 5% | Dimethicone | 5% | 85.3% | 8.6% | 0.10 | Good |
| Example 33 | Dry | Titanium oxide | 10 | Silicon aluminum | 5% 10% | Dimethicone | 15.0% | Polyglyceryl-3 polydimethylsiloxyethyl dimethicone | 5% | Dimethicone | 5% | 84.2% | 8.4% | 0.10 | Good |
| Example 34 | Dry | Titanium oxide | 10 | Silicon aluminum | 5% 10% | Triethoxycaprylylsilane | 22.0% | Sorbitan sesquiisostearate | 5% | C12-15 alkyl benzoate | 5% | 83.2% | 7.4% | 0.09 | Good |
| Example 35 | Dry | Titanium oxide | 30 | - | - | Dimethicone | 6.8% | PEG-9 polydimethylsiloxyethyl dimethicone | 5% | Dimethicone | 5% | 76.8% | 3.4% | 0.04 | Good |
| Example 36 | Wet | Titanium oxide | 8 | Aluminum | 13% | Stearic acid | 29.2% | Sorbitan sesquiisostearate | 50% | C12-15 alkyl benzoate | 5% | 91.2% | 9.8% | 0.11 | Good |
| Example 37 | Wet | Titanium oxide | 8 | Aluminum | 13% | Stearic acid | 29.2% | Sorbitan sesquiisostearate | 5% | C12-15 alkyl benzoate | 50% | 90.5% | 6.9% | 0.08 | Good |
| Comparative Example 1 | Wet | Titanium oxide | 15 | Aluminum | 10% | Stearic acid | 16.5% | - | - | - | - | 91.9% | 53.4% | 0.58 | Bad |
| Comparative Example 2 | Wet | Titanium oxide | 15 | Aluminum | 10% | Stearic acid | 16.5% | Polyhydroxystearic acid | 5% | - | - | 92.3% | 43.2% | 0.47 | Bad |
| Comparative Example 3 | Wet | Titanium oxide | 15 | Aluminum | 10% | Stearic acid | 16.5% | Polyhydroxystearic acid | 10% | - | - | 88.7% | 20.4% | 0.23 | Bad |
| Comparative Example 4 | Wet | Titanium oxide | 15 | Aluminum | 10% | Stearic acid | 16.5% | - | - | Hexyl laurate | 15% | 84.4% | 49.9% | 0.59 | Bad |

EP 4 714 424 A1

(continued)

| Powder composition | Processing method | Powder to be treated | | Metal oxide or metal hydroxide | | Hydrophobic treatment agent | | Surfactant | | Oil agent | | Transmittance | | | Evaluation |
|---|---|---|---|---|---|---|---|---|---|---|---|---|---|---|---|
| | | Kind | Particle diameter [nm] | Kind | Content | Kind | Content | Kind | Content | Kind | Content | 400 nm | 300 nm | 300/400 | |
| Comparative Example 5 | Wet | Titanium oxide | 15 | Aluminum | 10% | Stearic acid | 16.5% | Polyhydroxystearic acid | 55% | Hexyl laurate | 5% | 93.5% | 15.2% | 0.16 | Bad |
| Comparative Example 6 | Wet | Titanium oxide | 15 | Aluminum | 10% | Stearic acid | 16.5% | Polyhydroxystearic acid | 5% | Hexyl laurate | 55% | 95.6% | 12.1% | 0.13 | Bad |
| Comparative Example 7 | Dry | Titanium oxide | 10 | Silicon aluminum | 5% 10% | Dimethicone | 15.0% | - | - | - | - | 93.5% | 42.2% | 0.45 | Bad |
| Comparative Example 8 | Dry | Titanium oxide | 10 | Silicon aluminum | 5% 10% | Triethoxycaprylylsilane | 22.0% | - | - | - | - | 92.4% | 39.7% | 0.43 | Bad |

EP 4 714 424 A1

As shown in Table 1, in each of Examples 1 to 37, the 300 nm-transmittance is low, 10% or less, also in weak dispersion as in the dispersibility test, and ultraviolet ray shielding ability is excellent. Furthermore, in each of Examples 1 to 37, (300 nm-transmittance)/(400 nm-transmittance) is small, 0.2 or less, the 300 nm-transmittance is low, the ultraviolet ray shielding ability is high, and the 400 nm-transmittance is high, and transparency is high. Accordingly, it is seen that each of Examples 1 to 37 achieves both of high ultraviolet ray shielding ability and high transparency in a compatible manner. In each of Comparative Examples 1 to 8, the 300 nm-transmittance exceeds 10%, as compared with Examples, the ultraviolet ray shielding ability is inferior. In addition, in each of Comparative Examples 1 to 8, since the 300 nm-transmittance is high, a value of (300 nm-transmittance)/(400 nm-transmittance) is also high, both of the ultraviolet ray shielding ability and the transparency cannot be achieved in a compatible manner.

<Manufacturing of W/O type emulsified cosmetics>

Example 38

Put into a 150 mL-cup formed of polypropylene was 30.8 g of a mixture of oil phase raw materials shown below and while agitation was conducted at 500 rpm by using a high-speed emulsifying and dispersing machine "T.K.ROBOMICS" manufactured by PRIMIX Corporation, 7.0 g of the powder composition in Example 1 was added. Subsequently, an agitation speed was increased to 1500 rpm and agitation was conducted for 10 minutes. Thereafter, while agitation was continued, 32.2 g of a mixture of water phase raw materials shown below was added and agitation was conducted at 1500 rpm for 5 minutes, thereby preparing an emulsified cosmetic in Example 38.

Example 39

An emulsified cosmetic in Example 39 was prepared by using the powder composition in Example 14 in the same method as in Example 38.

Example 40

An emulsified cosmetic in Example 40 was prepared by using the powder composition in Example 17 in the same method as in Example 38.

Example 41

An emulsified cosmetic in Example 41 was prepared by using the powder composition in Example 25 in the same method as in Example 38.

Example 42

An emulsified cosmetic in Example 42 was prepared by using the powder composition in Example 32 in the same method as in Example 38.

Comparative Example 9

An emulsified cosmetic in Comparative Example 9 was prepared by using the powder composition in Comparative Example 1 in the same method as in Example 38.

Comparative Example 10

An emulsified cosmetic in Comparative Example 10 was prepared by using the powder composition in Comparative Example 4 in the same method as in Example 38.

Comparative Example 11

An emulsified cosmetic in Comparative Example 11 was prepared by using the powder composition in Comparative Example 7 in the same method as in Example 38.
Oil phase raw materials of the W/O type emulsified cosmetics were as follows.

23.8 g of isododecane: manufactured by Maruzen Petrochemical "Marukasol R"

3.5g of liquid paraffin: manufactured by MORESCO Corporation "MORESCO-WHITE P-70"

3.5 g of lauryl PEG-9 polydimethylsiloxyethyl dimethicone: manufactured by Shin-Etsu Chemical Co., Ltd. "KF-6038"

Water phase raw materials of the W/O type emulsified cosmetics were as follows.

22.4 g of ion-exchanged water

9.8 g of 1,3-butylene glycol

<Evaluation (sense of transparency) of cosmetics>

Immediately after 0.05 g of each of the emulsified cosmetics in Examples 38 to 42 and Comparative Examples 9 to 11 was applied to skin of forearms of 10 panelists, whiteness and a sense of transparency were visually evaluated under sunlight while a viewing angle was changed. In particular, blueness in a portion through which a vein looked blue was observed. Each thereof was left to stand for 5 minutes after each thereof was further applied and spread and after drying each thereof, a sense of transparency was evaluated. Each of the panelists cast one vote when the transparency of each of the emulsified cosmetics was higher than that in Comparative Example 9 with that in Comparative Example 9 regarded as a standard. Results are shown in Table 2.

When the emulsified cosmetics immediately after applying and spreading and after drying were compared, 8 panelists or more (10 panelists) evaluated that the sense of transparency in each of the emulsified cosmetics in Examples 38 to 42 was high and cast one vote, and the sense of transparency of each thereof in Examples 38 to 42 was evaluated as being higher than that of any of those in Comparative Examples 9 to 11.

<Evaluation (transmittance) of cosmetics>

By using an automatic bar coater with a wire bar (No. #6) attached, 1.0 g of each of the emulsified cosmetics in Examples 38 to 42 or Comparative Examples 9 to 11 was applied to polypropylene film (manufactured by RM TOHCELLO CO., LTD.: plain OPP sheet #40), thereby forming coated film (film thickness: 10 $\mu$m).

Total light transmittance of this coated film was measured by using a spectrophotometer (manufactured by Hitachi High-Tech Corporation "U-4100", using an integrating sphere) under the following conditions, and transmittance of 300 nm (ultraviolet ray shielding ability), 400 nm (transparency), and 450 nm (transparency) was read.

Measurement conditions were as follows.

Scanning speed: 300 nm/minutes

Sampling interval: 2 nm

Measurement wavelength: 250 nm to 700 nm

Results are shown in Table 2.

[Table 2]

| Emulsified cosmetic | Powder composition | Sense of transparency | | Transmittance | | |
|---|---|---|---|---|---|---|
| | | Immediately after applying and spreading | After drying | 300-nm ultraviolet ray shielding ability | 400-nm transparency | 450-nm transparency |
| Example 38 | Example 1 | 10 votes | 10 votes | 1.1% | 64.0% | 74.8% |
| Example 39 | Example 14 | 10 votes | 10 votes | 1.4% | 64.6% | 75.0% |
| Example 40 | Example 17 | 10 votes | 10 votes | 1.3% | 66.2% | 77.3% |
| Example 41 | Example 25 | 10 votes | 10 votes | 1.3% | 64.8% | 75.6% |
| Example 42 | Example 32 | 10 votes | 10 votes | 1.6% | 67.2% | 78.3% |
| Comparative Example 9 | Comparative Example 1 | Standard | Standard | 1.3% | 56.2% | 67.3% |
| Comparative Example 10 | Comparative Example 4 | 3 votes | 3 votes | 2.4% | 59.5% | 68.5% |

(continued)

| Emulsified cosmetic | Powder composition | Sense of transparency | | Transmittance | | |
|---|---|---|---|---|---|---|
| | | Immediately after applying and spreading | After drying | 300-nm ultraviolet ray shielding ability | 400-nm transparency | 450-nm transparency |
| Comparative Example 11 | Comparative Example 7 | 3 votes | 3 votes | 2.8% | 59.9% | 68.6% |

Since the transmittance of 300 nm (ultraviolet ray shielding ability) of each of the emulsified cosmetics in Examples 38 to 42 and Comparative Examples 9 to 11 was low, each of the emulsified cosmetics in Examples 38 to 42 and Comparative Examples 9 to 11 exhibited high ultraviolet ray shielding ability. Since the transmittance of each of 400 nm (transparency) and 450 nm (transparency) of each thereof in Examples 38 to 42 was higher than that in the emulsified cosmetic in Comparative Example 9 (standard), the transparency of each thereof in Examples 38 to 42 was high. Since the transmittance of each of 400 nm (transparency) and 450 nm (transparency) in each of the emulsified cosmetics in Comparative Examples 10 to 11 was equivalent to that in the emulsified cosmetic in Comparative Example 9 (standard), enhancement of the transparency was not recognized.

<Manufacturing of O/W type emulsified cosmetics>

Example 43

Put into a 200 mL-cup formed of polypropylene was 30.4g of a mixture of oil phase raw materials (except the powder composition in Example 1) shown in Table 3 and while agitation was conducted at 500 rpm by using a high-speed emulsifying and dispersing machine "T.K.ROBOMICS" manufactured by PRIMIX Corporation, and 9.6 g of the powder composition in Example 1was added. Subsequently, an agitation speed was increased to 5,000 rpm and agitation was conducted for 5 minutes. Subsequently, 40 g of a mixture of water phase raw materials shown in Table 3 was measured into a 150 mL-cup formed of polypropylene and while heating was conducted, by using the high-speed emulsifying and dispersing machine "T.K.ROBOMICS" manufactured by PRIMIX Corporation, agitation was conducted until PEG-100 hydrogenated castor oil was completely dissolved. Thereafter, while the water phase raw materials were agitated by a propeller, the oil phase raw materials were slowly added and agitation was conducted at 1,500 rpm for 3 minutes by using a homomixer, thereby preparing an emulsified cosmetic in Example 43.

[Table 3]

| | Display name | Blending proportion (weight%) |
|---|---|---|
| Water phase | Water | 36.86% |
| | BG (manufactured by KH Neochem Co., Ltd., 1,3-BG) | 4.00% |
| | Ethanol | 4.00% |
| | (manufactured by Sigma-Aldrich Co. LLC, ethanol) | |
| | Silica (manufactured by TAYCA CORPORATION, TMS-05DCB) | 3.00% |
| | PEG-100 hydrogenated castor oil (manufactured by Nikko Chemicals Co., Ltd., NIKKOL HCO-100) | 1.20% |
| | Polysorbate 80 (manufactured by Nikko Chemicals Co., Ltd., NIKKOL TO-10V) | 0.24% |
| | Hydroxyethyl acrylate/sodium acryloyldimethyl taurate copolymer (manufactured by SEIWA KASEI CO., LTD., SEPINOV EMT-10) | 0.30% |
| | Hydroxypropyl methylcellulose stearoxy ether (manufactured by Daido Chemical Industry Co.,Ltd., SANGELOSE 90L) | 0.30% |
| | Xanthan gum (manufactured by MP Gokyo Food & Chemical Co., Ltd., Echo Gum T) | 0.10% |

(continued)

| | Display name | Blending proportion (weight%) |
|---|---|---|
| Oil phase | Hydrogenated polyisobutene (manufactured by NOF CORPORATIO, PERLEAM 4) | 34.00% |
| | Powder composition in Example 1 | 12.00% |
| | Polyhydroxystearic acid (manufactured by The Nisshin OilliO Group, Ltd., SALACOS HS-6C) | 0.50% |
| | Polyglyceryl-5 polyricinoleate (manufactured by Taiyo Kagaku Co.,Ltd., Sunsoft 818R-C) | 0.50% |
| | Caprylyl methicone (manufactured by The Dow Chemical Co., DOWSIL SS-3408) | 3.00% |

Examples 44 to 46 and Comparative Examples 12 to 14

An emulsified cosmetic in each of Examples 44 to 46 and Comparative Examples 12 to 14 was prepared by the same method as in Example 43 except that the powder composition in Example 1 was changed to a powder composition shown in Table 4.

<Evaluation of emulsified state>

External appearance of the emulsified cosmetics in Examples 43 to 46 and Comparative Examples 12 to 14 immediately after the emulsified cosmetics were prepared was checked and those which were capable of being emulsified were evaluated as "Good" and those which were not capable of being emulsified were evaluated as "Bad". In addition, the emulsified cosmetics in Example 43 to 46 and Comparative Examples 12 to 13 which were capable of being emulsified immediately after preparation were stored for 2 months in a thermal insulating storage whose temperature was set at 50°C, and thereafter, external appearance (separation of an oil phase and a water phase, presence/absence of agglomerate) thereof was checked and those whose each emulsified state was not changed from that immediately after preparation was evaluated as "Good" and those whose each emulsified state was changed from that immediately after preparation was evaluated as "Bad". These evaluation results are shown in Table 4.

[Table 4]

| Emulsified cosmetic | Powder composition | Emulsified state | |
|---|---|---|---|
| | | Immediately after preparation | After storage for 2 months at 50°C |
| Example 43 | Example 1 | Good | Good |
| Example 44 | Example 25 | Good | Good |
| Example 45 | Example 27 | Good | Good |
| Example 46 | Example 32 | Good | Good |
| Comparative Example 12 | Comparative Example 1 | Good | Bad (syneresis) |
| Comparative Example 13 | Comparative Example 4 | Good | Bad (syneresis) |
| Comparative Example 14 | Comparative Example 7 | Bad | - |

Each of those in Examples 43 to 46 formed the emulsified state and the uniform emulsified state was maintained also after the storage for 2 months at 50°C. Although each of those in Comparative Examples 12 to 13 formed the emulsified state immediately after preparation, each of those in Comparative Examples 12 to 13 was not being capable of maintaining a uniform emulsified state after the storage for 2 months at 50°C. That in Comparative Example 14 was not capable of forming an emulsified state at the time of immediately after preparation, resulting in inferior dispersibility.

As described above, since the powder composition of the present invention has extremely high dispersibility, there is little agglomeration when the powder composition thereof is blended into an inner oil phase, an O/W type emulsified cosmetic having high stability can be provided. In addition, it was confirmed that regardless of dosage forms, this high dispersibility contributes to high ultraviolet ray protective effect and exhibition of transparency for not only O/W type emulsified cosmetics but also W/O type emulsified cosmetics and furthermore, the powder composition thereof is effective also for saving of energy and shortening of time which are required for dispersion.

The described embodiment and examples are to be considered in all respects only as illustrative and not restrictive. It is intended that the scope of the invention is, therefore, indicated by the appended claims rather than the foregoing description of the embodiment and examples and that all modifications and variations coming within the meaning and equivalency range of the appended claims are embraced within their scope.

**Claims**

1.  A powder composition including:

    titanium oxide powder as powder to be treated;
    at least one hydrophobic treatment agent selected from the group consisting of a fatty acid, silicone, and a silane coupling agent;
    a surfactant; and
    an oil agent, wherein a content of the surfactant is 0.1% or more and 50% or less of a weight of the powder to be treated, and
    a content of the oil agent is 0.1% or more and 50% or less of the weight of the powder to be treated.

2.  The powder composition according to claim 1, wherein a content of the hydrophobic treatment agent is 7% or more and 30% or less of the weight of the powder to be treated.

3.  The powder composition according to claim 1 or 2, wherein total light transmittance at a wavelength of 300 nm is 10% or less and a value of (300 nm-transmittance)/(400 nm-transmittance) is 0.2 or less.

4.  A cosmetic including the powder composition according to any one of claims 1 to 3.

5.  A method for manufacturing a powder composition comprising:

    an alkalinizing step of adding an alkali metal hydroxide to an aqueous dispersion of a hydrous titanium oxide and obtaining alkali metal titanate;
    an acidizing step of adding hydrochloric acid to an aqueous dispersion of the alkali metal titanate and obtaining a titanium oxide including rutile type crystals; and
    a mixing step of mixing at least, at least one hydrophobic treatment agent selected from the group consisting of a fatty acid, silicone, and a silane coupling agent, a surfactant, and an oil agent to the titanium oxide obtained at the acidizing step.

6.  The method for manufacturing a powder composition according to claim 5 wherein, the mixing step includes adding of the surfactant and the oil agent after mixing the titanium oxide and the hydrophobic treatment agent.

7.  The method for manufacturing a powder composition according to claim 5 wherein, the mixing step is a step of mixing at least a metal oxide or a metal hydroxide, the hydrophobic treatment agent, the surfactant, and the oil agent with the titanium oxide obtained at the acidizing step.

8.  The method for manufacturing a powder composition according to claim 7, wherein the mixing step includes adding the surfactant and the oil agent after mixing the titanium oxide, the metal oxide or the metal hydroxide, and the hydrophobic treatment agent.

9.  The method for manufacturing a powder composition according to claim 7, wherein the mixing step includes adding the hydrophobic treatment agent, the surfactant, and the oil agent after mixing the titanium oxide and the metal oxide or the metal hydroxide.

# INTERNATIONAL SEARCH REPORT

| International application No. |
|---|
| **PCT/JP2024/018031** |

| A. | CLASSIFICATION OF SUBJECT MATTER |
|---|---|

*A61K 8/29*(2006.01)i; *A61Q 17/04*(2006.01)i; *C01G 23/053*(2006.01)i
FI:    A61K8/29; A61Q17/04; C01G23/053

According to International Patent Classification (IPC) or to both national classification and IPC

| B. | FIELDS SEARCHED |
|---|---|

Minimum documentation searched (classification system followed by classification symbols)

A61K8/29; A61Q17/04; C01G23/053

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2016-222594 A (TEIKA CORP.) 28 December 2016 (2016-12-28) claims, paragraphs [0029]-[0043], [0047], [0048], example 5, fig. 8 | 1-4 |
| Y | | 5-9 |
| Y | JP 11-171753 A (TEIKA CORP.) 29 June 1999 (1999-06-29) claims, paragraphs [0001], [0008], [0011], examples 1-3 | 5-9 |
| A | JP 2016-222589 A (TEIKA CORP.) 28 December 2016 (2016-12-28) whole document | 1-9 |
| A | JP 2011-93826 A (MIYOSHI KASEI, INC.) 12 May 2011 (2011-05-12) whole document | 1-9 |
| A | JP 7-247119 A (ISHIHARA SANGYO KAISHA, LTD.) 26 September 1995 (1995-09-26) whole document | 1-9 |
| P, A | WO 2023/199644 A1 (DNP FINE CHEMICALS CO., LTD.) 19 October 2023 (2023-10-19) whole document | 1-9 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 July 2024** | **23 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/018031**

| Patent document cited in search report | | | Publication date (day/month/year) | Patent family member(s) | Publication date (day/month/year) |
|---|---|---|---|---|---|
| JP | 2016-222594 | A | 28 December 2016 | (Family: none) | |
| JP | 11-171753 | A | 29 June 1999 | (Family: none) | |
| JP | 2016-222589 | A | 28 December 2016 | (Family: none) | |
| JP | 2011-93826 | A | 12 May 2011 | (Family: none) | |
| JP | 7-247119 | A | 26 September 1995 | (Family: none) | |
| WO | 2023/199644 | A1 | 19 October 2023 | (Family: none) | |

Form PCT/ISA/210 (patent family annex) (July 2022)

**EP 4 714 424 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009102236 A

- JP 2010215602 A